# EUROPEAN PATENT SPECIFICATION

(11) **EP 0 690 126 B1**
(45) Date of publication and mention of the grant of the patent: **28.11.2001**
(21) Application number: 95110468.6
(22) Date of filing: 13.06.1988
(51) Int. Cl.: C12N 15/12, A61K 38/37, G01N 33/50, C12N 15/62, C07K 14/755, C12P 21/02, C12N 5/10, C12N 1/19, C12N 1/21

(54) **Novel proteins with factor VIII activitiy: process for their preparation using genetically-engineered cells and pharmaceutical compositions containing them**
Proteine mit Faktor VIII Aktivität, Verfahren zu deren Herstellung unter Verwendung von genetisch modifizierten Zellen und diese enthaltende Zusammensetzungen
Protéines à activité de facteur VIII, procédé pour leur production utilisant des cellules modifiées par génie génétique et compositions pharmaceutiques les contenant

(30) Priority: 12.06.1987 EP 87201121
(43) Date of publication of application: 03.01.1996
(62) Divisional of application: 88201209.9
(73) Proprietor: Baxter Aktiengesellschaft, 1221 Wien (AT)
(72) Inventor: Van Ooyen, Albert Johannes Joseph, NL-2271 AR Voorburg (NL); Pannekoek, Hans, c/o Centraal Laboratorium CLB, NL-1066 CX Amsterdam (NL); Verbeet, Martinus P., NL-1066 CX Amsterdam (NL); Van Leen, Robert Willem, NL-3065 NG Rotterdam (NL)
(74) Representative: Davies, Jonathan Mark

(56) References cited:
- EP-A- 0 160 457
- EP-A- 0 232 112
- EP-A- 0 253 455
- EP-A- 0 265 778
- WO-A-86/06101
- WO-A-87/07144
- WO-A-88/00831
- JOURNAL OF BIOLOGICAL CHEMISTRY (MICROFILMS), vol. 261, no. 27, September 1986 MD US, pages 12574-12578, BURKE R. ET AL. 'The functional domains of coagulation factor VIII:C'
- BIOCHEMISTRY, vol. 25, no. 26, December 1986 EASTON, PA US, pages 8343-8347, EATON D. L. ET AL. 'Construction and characterization of an active factor VIII variant lacking the central one-third of the molecule'
- PROCEEDINGS OF THE NATIONAL ACADEMY OF SCIENCES OF USA, vol. 83, August 1986 WASHINGTON US, pages 5939-5942, TOOLE J. ET AL. 'A large region (95 kDa) of human factor VIII is dispensable for in vitro procoagulant activity'

## Description

### Technical Field

The invention relates to novel proteins having Factor VIII activity and methods for their preparation using genetically engineered cell-lines and microorganisms. This application is a divisional application based on European Patent Application No. 88201209.9.

### Background

Hemophilia A is a sex-linked bleeding disorder characterized by a deficiency in Factor VIII, an essential element in the blood coagulation cascade. The disease occurs in about 0.01% of the male population. Hemophilia A can be treated by administering Factor VIII-containing blood plasma obtained from healthy donors. This treatment has several disadvantages however. The supply of Factor VIII is limited and very expensive; the concentration of Factor VIII in blood is only about 100 ng/ml and the yields using current plasma fractionation methods are low. Since the source of Factor VIII is pooled donor blood, the recipient runs a high risk of acquiring various infectious diseases, including those caused by hepatitis non-A, non-B, hepatitis B or AIDS viruses which may be present in the donor blood. In addition, recipients may develop antibodies against the exogenous Factor VIII, which can greatly reduce its effectiveness.

Factor VIII comprises three regions, an N-terminal region, the so-called "A1A2-domain"; a central region, the so-called "B domain"; and a C-terminal region comprising the A3, C1 and C2 domains. The AlA2-domain and the C-terminal region are believed essential for clotting activity. Factor VIII circulates in the blood combined with a protein, the von Willebrand factor (vWf), which is believed to protect the sensitive Factor VIII against early degradation.

Factor VIII is obtained in unsatisfactorily low yields when produced by known recombinant DNA processes. Moreover the proteins appear not to be present as an intact chain, hence products are difficult to isolate and to purify and consequently the costs are high. It is therefore desirable to develop an efficient way to produce large quantities of compounds having Factor VIII activity, the compounds preferably having decreased immunogenic activity.

### Relevant Literature

Molecular cloning of Factor VIII cDNA obtained from human mRNA and the subsequent production of proteins with Factor VIII activity in mammalian, yeast and bacterial cells has been reported. See WO 85/01961, EP 0160457, EP 0150735, EP 0253455. A method for producing proteins with Factor VIII activity using transformed microorganisms is disclosed in EP 0253455. European patent applications EP 0150735 and EP 0123945 and Brinkhous et al. (1985) disclose Factor VIII activity in proteolytic cleavage products of Factor VIII. A complex of two proteolytic cleavage products of Factor VIII, a 92 kDa and an 80 kDa polypeptide exhibits enhanced Factor VIII activity. (Fay et al., Biochem. Biophys. Acta (1986) 871:268-. 278; Eaton et al Biochemistry (1986) 25:505-512).

Eaton et al., Biochemistry (1986) 25:8343-8347 disclose that a polypeptide in which 766 amino acids (797-1562) have been deleted from the central region retains Factor VIII activity. Moreover, mammalian cells transformed with a vector comprising DNA encoding this deletion polypeptide had a higher production level than cells transformed with a vector comprising DNA encoding the full length polypeptide.

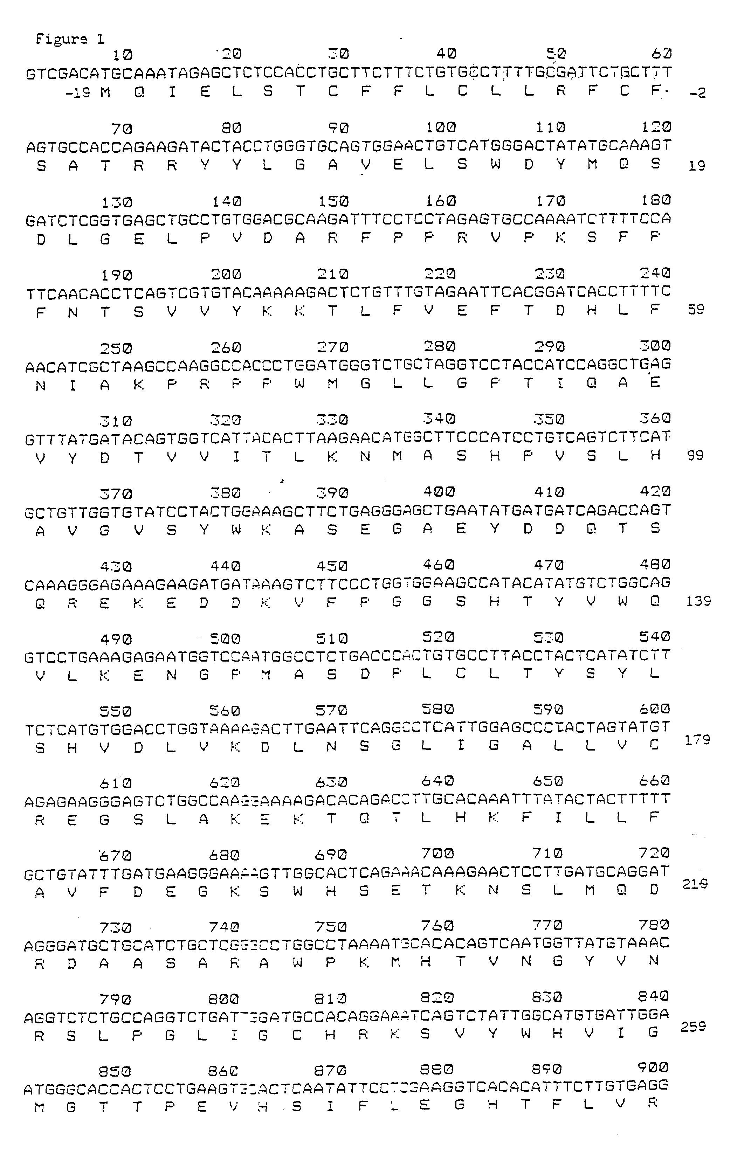

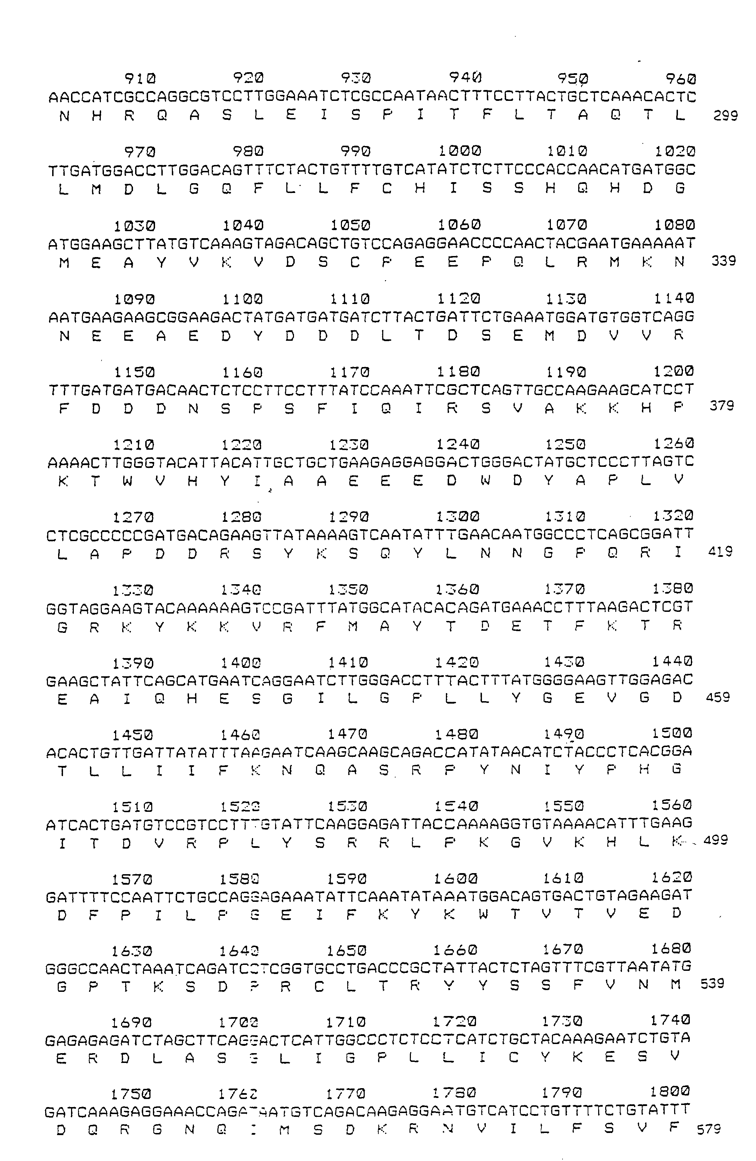

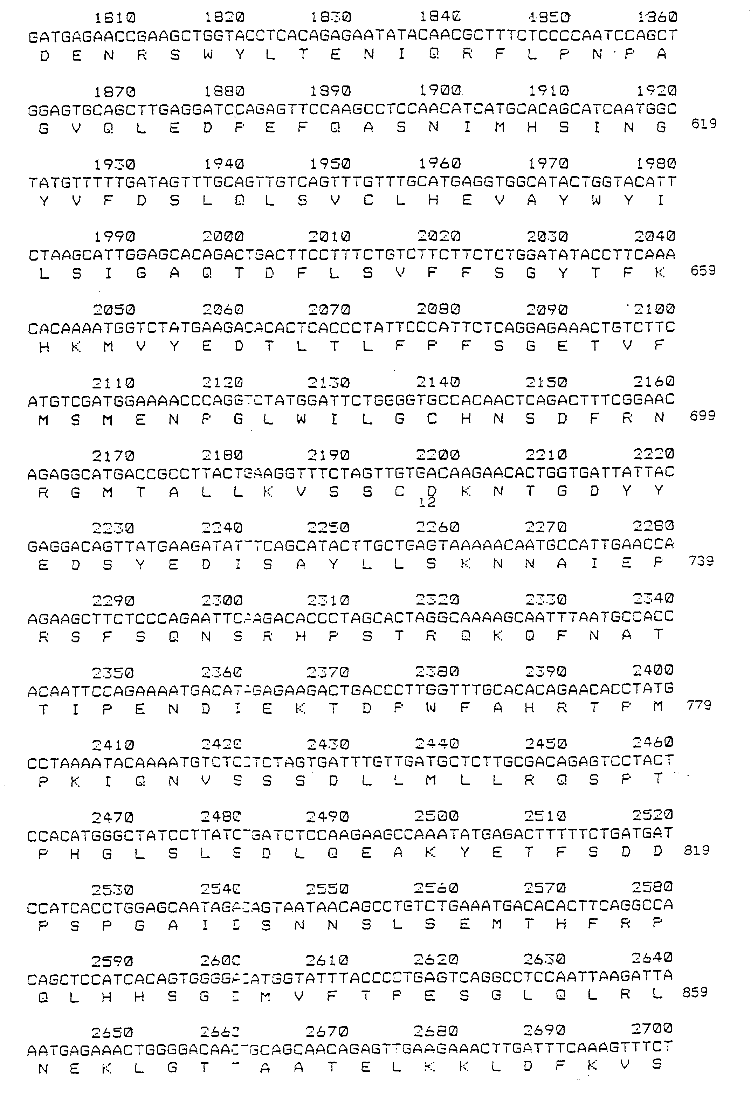

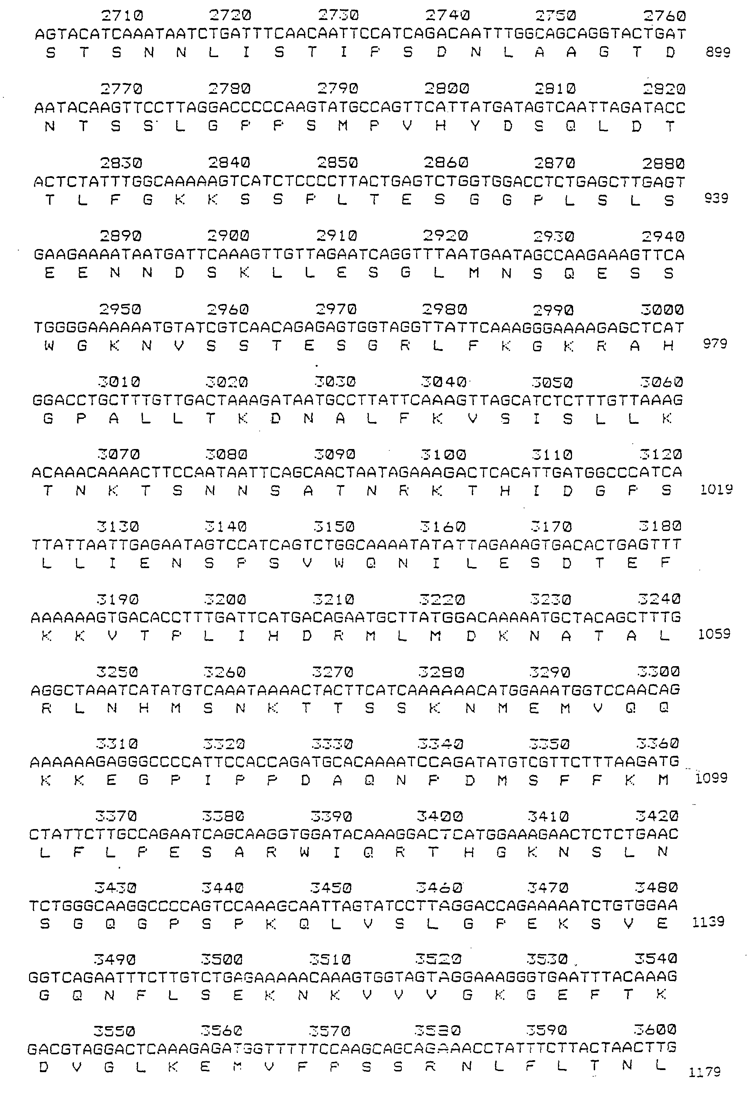

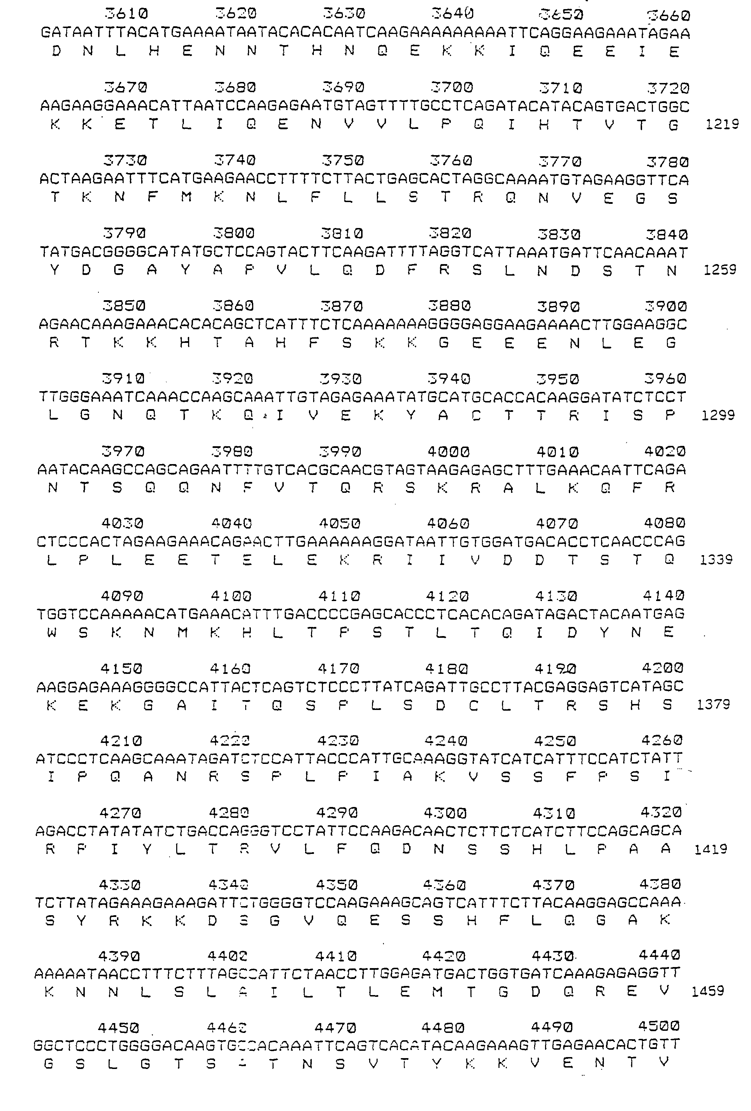

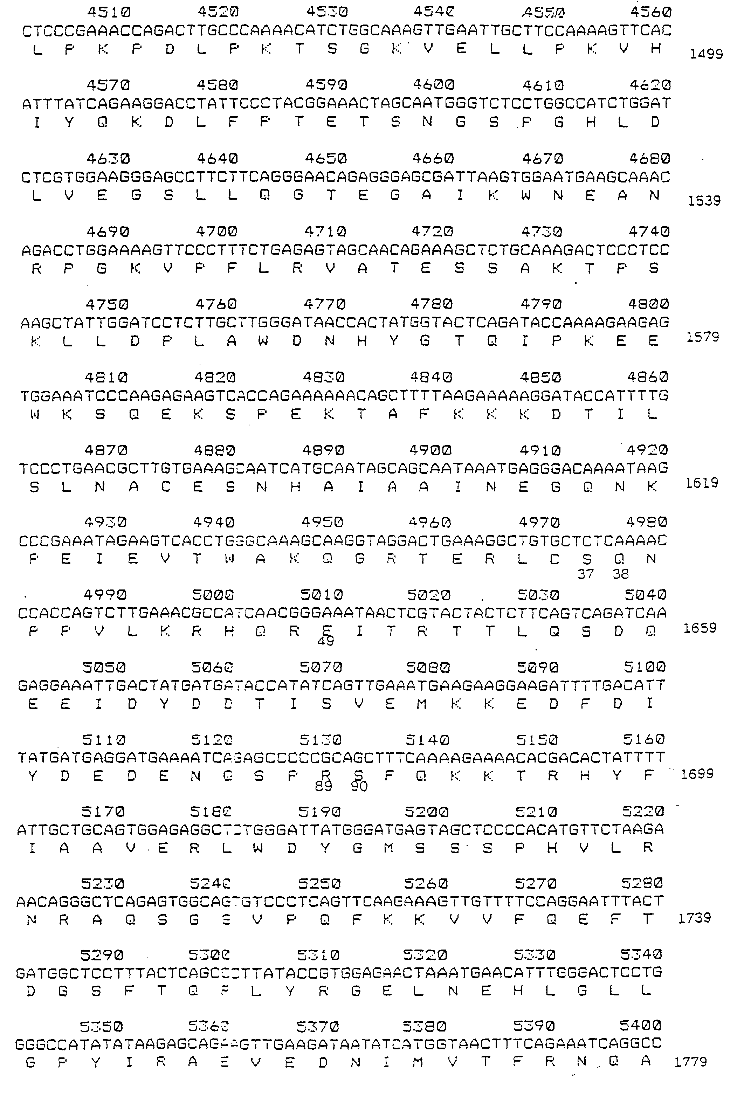

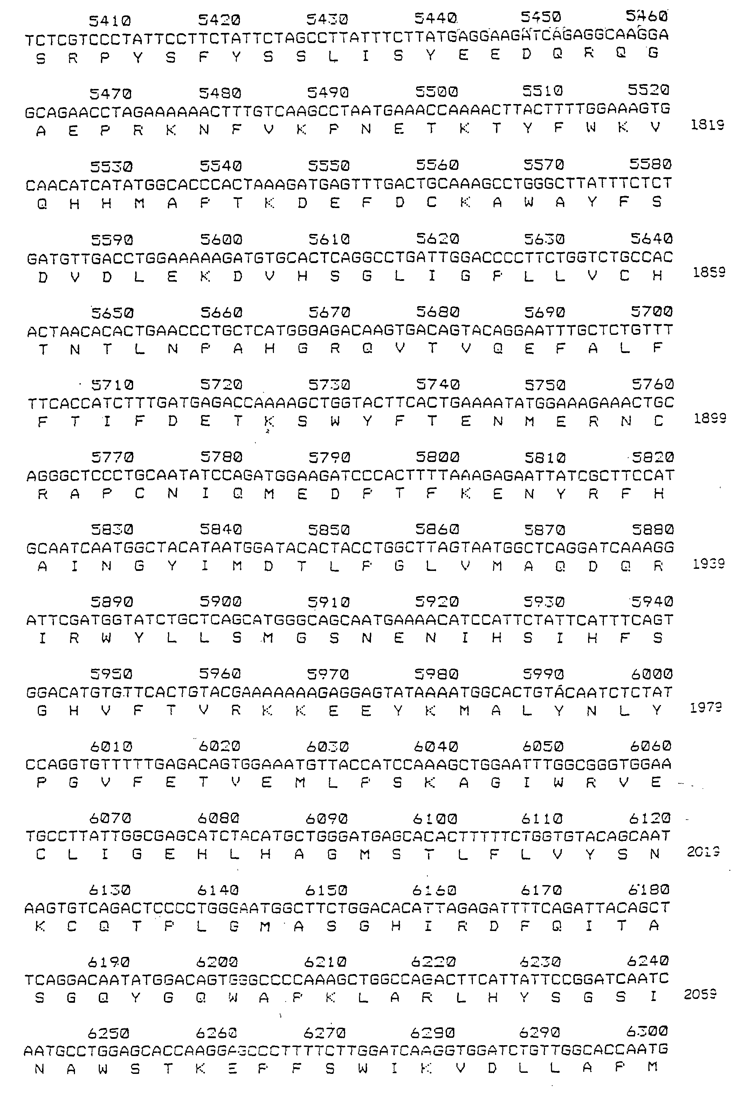

PCT application WO 86/06101 discloses that recombinant Factor VIII proteins with deletions of up to 880 amino acids in the central region exhibit Factor VIII activity. The largest deletion stretches from Thr-760 through Asn-1639 (numbering according to Figure 1). The host cells for preparation of the recombinant Factor VIII included mammalian cells, including Chinese hamster ovary cells.

US Patent 4,868,112 discloses DNA encoding a truncated Factor VIII protein with procoagulant activity, having a deletion of at least 581 amino acids within the region between Arg-740 and Ser-1690 of the full length sequence (numbering according to Figure 1).

### SUMMARY OF THE INVENTION

Novel compositions, together with expression vectors and methods for their preparation are provided, which comprise derivatives of Factor VIII. The compositions are prepared by transforming a host cell, preferably a mammalian cell, with an expression vector comprising a DNA sequence encoding a Factor VIII congener, growing the transformed host cell to express the exogenous DNA sequence, and recovering the resultant Factor VIII derivative from a cell lysate or from conditioned growth medium.

### BRIEF DESCRIPTION OF THE DRAWINGS

Figure 1 shows the Factor VIII cDNA insert of pCLB89 including the amino acid sequence Ala-1 through Tyr-2332 of Factor VIII and its signal sequence M(-19) through S(-1) with the corresponding sequence of nucleotides. F-973 is encoded by the codon TTC.

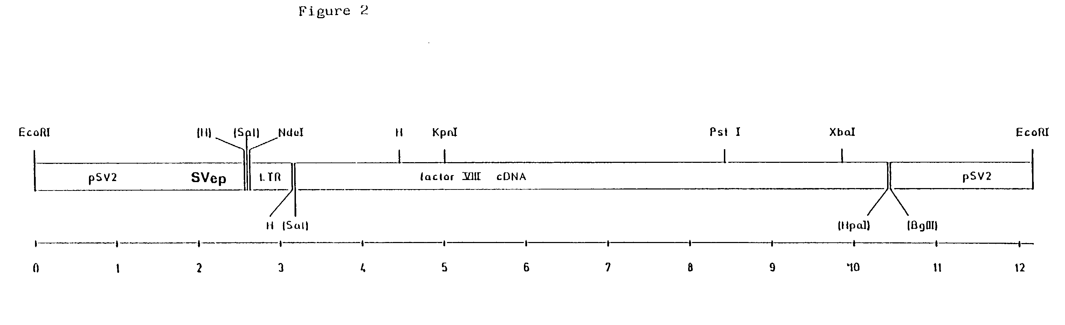

Figure 2 shows the structure of expression vector pCLB201 encoding the full-length Factor VIII protein. The circular plasmid pCLB201 is shown beginning with the EcoRI position within the 4217 bp HindIII-BglII fragment derived from plasmid pSV2 (Gorman, 1985 DNA Cloning (Ed. Glover) IRL Press, pp. 143-169; Mulligan and Berg, Proc. Natl. Acad. Sci. USA (1981) 78:2072). The early SV40-promoter region is indicated: SVep. The vector also contains a NdeI-HindIII fragment derived from plasmid pRSV-neo (Gorman, 1985, supra) bearing the Rous Sarcoma Virus-Long Terminal Repeat (LTR) inserted into a SalI site. The full-length Factor VIII is encoded by a SalI-HpaI fragment of 7440 bp and indicated: Factor VIII cDNA (H = HindIII, Sal = SalI). The restriction endonuclease cleavage sites lost during construction of pCLB201 are enclosed in parenthesis. The size of the plasmid is given in kilobase pairs (kb).

Figure 3 shows vectors for transient expression of deletion mutant Factor VIII proteins:
a. The landmarks of the pSV2-derived vector: two tandemly situated promoters: the SV40 early transcription promoter (SVep) and the Rous Sarcoma Virus-Long Terminal Repeat (RSV-LTR); the capping site (Capsite) and 5' end of the messenger RNA (mRNA); the cDNA insert bearing the full-length Factor VIII coding region with the start codon (ATG), the open reading frame and the stop codon (TGA); the 3' non-coding region of the mRNA with a short intron and the polyadenylation signal (polyA) derived from SV40 DNA (compare to Figure 2).
b. The 7440 bp (base pairs) fragment SalI-HpaI bearing the full-length Factor VIII coding cDNA is depicted. The start and stop codons of the reading frame are indicated. Restriction endonuclease sites within the full-length cDNA involved in the mutagenesis for the construction of pCLB202 and pCLB203 are shown.
c. Map of the Factor VIII protein. The 19 amino acid long signal peptide and the domain or repeat structure of plasma Factor VIII are shown. A1, A2 and A3 are homologous amino acid sequences. B is a unique region, whereas C1 and C2 are again homologous (Vehar et al., 1984). The amino acid positions bordering the A and C repeats are given. Below the map the cleavage sites of the proteolytic enzymes, that process Factor VIII, i.e., activated protein C (APC), thrombin (IIa), activated coagulation factor X (Xa), and a trypsin-like protease (indicated with "?") are indicated by arrows. Factor Xa is thought to cleave the IIa- and APC-cleavage sites also shown (Eaton et al., Biochemistry (1986) 25:8343-8347; Fay et al., Biochem. Biophys. Acta (1986) 871:268-278). Their cleavage sites are given. The B region contains multiple cleavage sites.
d. The subunit structure of activated Factor VIII. Factor VIIIa subunits of 92 kDa and 80 kDa are indicated as 92k and 80k, respectively. Their amino terminal and carboxy terminal amino acid positions within the full-length sequence are indicated.
e. The structure of the cDNA and protein of pCLB202 containing a direct fusion between the PstI and BamHI site indicated in b. The resulting protein is indicated containing a peptide bond between Ala-867 and Asp-1563. The total length of the protein is 1637 amino acids. (The landmarks of the repeat border positions and specific proteolytic cleavage sites are as depicted; see above.)
f. Embodiment of the invention: The structure of the cDNA and protein pCLB203
   containing a MluI-linker sequence coding for four extra amino acids that bridge the MaeIIIsite and HgiAT site; as indicated under b. The total length of the protein is 1411 amino acids.
   (The landmarks of the repeat border positions and specific proteolytic cleavage sites are depicted; see above.)

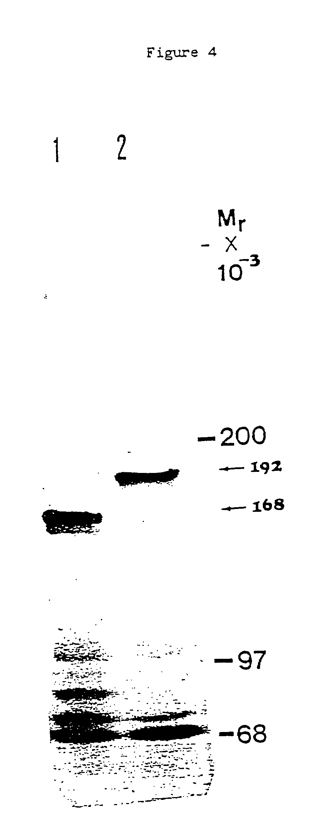

Figure 4 shows the results of a molecular weight (size) determination of several deletion mutant Factor VIII proteins using immunoprecipitation and electrophoresis.

The autoradiograph shows the lanes for the proteins made with the vectors pCLB202 (lane 2) and pCLB203 (according to the invention) (lane 1). The molecular weight markers are as indicated.

### DESCRIPTION OF THE SPECIFIC EMBODIMENTS

In accordance with the subject invention, novel DNA constructs and novel compositions comprising host cells producing polypeptides having Factor VIII activity are provided. The polypeptides having Factor VIII activity are deletion mutant proteins of Factor VIII in which substantially all of the central region or "B domain" as well as a portion of the 92 kilo-Dalton (kDa) region has been deleted. Plasmid constructs comprising a DNA sequence encoding deletion polypeptides having Factor VIII activity are used to transform a host cell. The host cell is then grown to express the gene. The host cell may be either a eukaryotic or a prokaryotic cell.

Human Factor VIII has the sequence shown in Figure 1. Single letter abbreviations for the amino acids are used, and have the following meaning: A = alanine; R = arginine; N = asparagine; D = aspartic acid; C = cysteine; Q = glutamine; E = glutamic acid, G = glycine; H = histidine; I = isoleucine; L = leucine; K = lysine; M = methionine; F = phenylalanine; P = proline; S = serine; T = threonine; W = tryptophan; Y = tyrosine; and V = valine. The numbering of the amino acid sequence starts with A-1, the first amino acid after the 19 amino acid signal sequence. The last amino acid-of Factor VIII is Y-2332. This numbering is used throughout the specification.

The subject matter and scope of the present invention is defined in the appended claims.

### Preparation of Congeners of Factor VIII

Factor VIII congeners may be obtained in a variety of ways. They may be obtained by proteolytic cleavage of the full-length Factor VIII into three regions, preferably by cleavage before Cys-711 and Gln-1638 followed by truncating the amino or carboxyl terminus of the Ala-1 throuch Ser-710 sequence at least one amino acid at a time and/or truncating the amino or carboxyl terminus of the sequence Gln-1638 through Tyr-2332 at least one amino acid at a time. The polypeptide fragments obtained may then be fused directly or via a central linking group, or the fragments may be combined in a composition to provide for Factor VIII activity.

Factor VIII congeners can also be prepared by recombinant DNA techniques. Techniques used in isolating the Factor VIII gene are known in the art, including synthesis, isolation from genomic DNA, preparation from cDNA, or combinations thereof. The various techniques for manipulation of the genes are well known, and include restriction, digestion, resection, ligation, in vitro mutagenesis, primer repair, and poly linkers and adapters, and the like. See Maniatis et al., Molecular Cloning, Cold Spring Harbor Laboratory, Cold Spring Harbor, New York, 1982.

Generally the method comprises preparing a genomic library from cells which synthesize Factor VIII. To enhance the likelihood of identifying the correct sequence, a cDNA library from cells which do not produce Factor VIII may be used to cross-hybridize. An assay for the expression of Factor VIII using restriction fragments inserted into a prokaryotic expression vector such as pTZ 18 or 19 and screening with antibodies for Factor VIII to detect a cross-reactive peptide fragment or the like can be used.

Once a complete gene has been identified, either as cDNA or chromosomal DNA, the desired deletions in the structural gene can be made in several ways. Deletions may be made by enzymatically cutting the full length Factor VIII cDNA followed by modification and ligation of the purified fragments or by site-directed mutagenesis, especially by loop-out mutagenesis as described by Kramer et al., Nucl. Acids Res. (1984) 12:9441-9456. The gene thus obtained may then be manipulated in a variety of ways well known in the art to provide for expression.
Both prokaryotic and eukaryotic hosts may be employed, which may include bacteria, yeast, mammalian cells, for example, CHO cells, C127 cells, human "293" cells, myeloma cells, or specialized cells such as liver cells, and COS cells. Therefore where the gene is to be expressed in a host which recognizes the wild-type transcriptional and translational regulatory regions of Factor VIII, the entire gene with its wild-type 5', and 3'-regulatory regions may be introduced into an appropriate expression vector. Various expression vectors exist employing replication systems from mammalian viruses, such as simian virus 40, Epstein-Barr virus, bovine papalloma virus, vaccinia virus, etc.

Where the gene is to be expressed in a host which does not recognize the naturally occurring wild-type transcriptional and translational regulatory regions, further manipulation will be required. Conveniently a variety of 3'-transcriptional regulatory regions are known and may be inserted downstream from the stop codons. The non-coding 5' region upstream form the structural gene may be removed by endonuclease restriction, Bal31 resection, or the like. Alternatively, where a convenient restriction site is present near the 5' terminus of the structural gene, the structural gene may be restricted and an adapter employed for linking the structural gene to the promoter region, where the adapter provides for the lost nucleotides of the structural gene.

Various strategies may be employed for providing a foreign expression cassette, which in the 5'-3'-direction of transcription has a transcriptional regulatory region and a translational initiation region, which may also include regulatory sequences allowing for the induction of regulation; an open reading frame encoding the full length Factor VIII or a congener of Factor VIII, including the deletion mutant proteins, desirably including a secretory leader sequence recognized by the proposed host cell; and trans-lational and transcriptional termination regions. The expression cassette may additionally include at least one marker gene. The initiation and termination regions are functional in the host cell, and may be either homologous (derived from the original host), or heterologous (derived from a foreign source) or synthetic DNA sequences. The expression cassette thus may be wholly or partially derived from natural sources, and either wholly or partially derived from sources homologous to the host cell, or heterologous to the host cell. The various DNA constructs (DNA sequences, vectors, plasmids, expression cassettes) of the invention are isolated and/or purified, or synthesized and thus are not "naturally occurring".

For optimal gene expression, the nucleotide sequences surrounding the translational initiation codon ATG have been found to be important in animal cells. For example, Kozak, Microbiol. Reviews (1983) 47:1-45, has studied extensively the effect of these regions on the expression of polypeptides such as insulin in COS cells. Thus it may be necessary to modify the nucleotide sequences surrounding the initiation codon. This can bed done by site-directed mutagenesis or by fusing the exogenous gene to the initiation region of a highly expressed gene.

Illustrative transcriptional regulatory regions or promoters include, for bacteria, the beta-gal promoter, amylase promoter, lambda left and right promoters, trp and lac promoters, trp-lac fusion promoter, etc.; for yeast, glycolytic enzyme promoters, such as ADH-1 -2 promoters, PGK promoter, and lactase promoter and the like; for mammalian cells, SV40 early and later promoters, cytomegalovirus (CMV) promoter, beta-actine promoter, adenovirus major late promoters and the like.

In eukaryotic cells, regulatory sequences can include, for example, the cytomegalovirus enhancer sequence which can be fused to a promoter sequence such as the SV40 promoter, forming a chimeric promoter, or inserted elsewhere in the expression cassette, preferably in close proximity to the promoter sequence.
Expression of the structural gene can also be amplified by, for example, ligating in tandem a gene for a dominant amplifiable genetic marker 5' or 3' to the structural gene and growing the host cells under selective conditions. An example of an amplifiable gene is the gene for dihydrofolate reductase (dhfr), the expression of which may be increased in cells rendered resistant to methotrexate (mtx), a folate antagonist. Of interest for expression in mammalian cells such as COS cells are expression cassettes capable of expressing Factor VIII or congeners thereof which employ a metallothionein promoter, or the SV40 early transcription unit transcription promoter (SVep), particularly the Rous Sarcoma Virus-Long Terminal Repeat (RSV-LTR) promoter in tandem with the SVep promoter. Examples of promoters and promoter/enhancer combinations which can be used in C127 cells in combination with expression vectors according to example 7 has been described in e.g. Hurwitz et al., Nucl. Acids Res. (1987) 15:7137-7153. For optimal expression the introduction of an intron into the expression cassette, may be beneficial. An intron in the 5'-part of the mRNA is preferred.

In addition, a fused gene may be prepared by providing a 5'-sequence to the structural gene which encodes a secretory leader and a processing signal. For secretion of Factor VIII polypeptides the naturally occurring Factor VIII leader sequence is preferably used, but other signal sequences including secretory leaders of penicillinase, alfa-factor, immunoglobulin, T-cell receptors, outer membrane proteins, serum albumin, tissue plasminogen activator, insulin, digestive tract enzymes, and the like may also be used. By fusion of a secretory leader in proper reading frame with a structural gene for Factor VIII or a congener thereof, the mature Factor VIII may be secreted into the culture medium.

The termination region may be derived from the 3' region of the gene from which the initiation region was obtained or from a different gene. A large number of termination regions are known and have been found to be satisfactory in a variety of hosts from the same and different genera and species. The termination region may include sequences for the proper processing of the mRNA in a mammalian cell: i.e., a small intron and a polyadenylation signal.

During the construction of the expression cassette, the various DNA fragments will usually be cloned in an appropriate cloning vector, which allows for expansion of the DNA, modification of the DNA or manipulation by joining or removing of the sequences, linkers, or the like. Normally, the vectors will be capable of replication in at least a relatively high copy number in E. coli. A number of vectors are readily available for cloning, including such vectors as pBR322, pML2, pUCT-pUC19, pSP64, pSP65, pSP18, pSP19, pTZ18 and pTZ18R, pTZ19 and pTZ19R and the like.

The cloning vectors are characterized as having an efficient origin of replication at least functional in E. coli. Also the cloning vector will have at least one unique restriction site, usually a plurality of unique restriction sites and may also include multiple restriction sites, particularly two of the same restriction sites for substitution. In addition, the cloning vector will have one or more markers which provide for selection for transformation. The markers will normally provide for resistance to cytotoxic agents such as antibiotics, heavy metals, toxins or the like, complementation of an auxotrophic host, or immunity to a phage. By appropriate restriction of the vector and cassette, and, as appropriate, modification of the ends, by chewing back or filling in overhangs, to provide for blunt ends, by addition linkers, by tailing, complementary ends can be provided for ligation, and joining of the vector to the expression cassette or a component thereof.

In some instances, a shuttle vector will be employed where the vector is capable of replication in different hosts requiring different replication systems. Preferably a simian virus 40 (SV40) origin of replication is employed which allows the plasmid to be propagated in COS-1, while the Bovine Papilloma Virus (BPV-1) genome is used for the episomal maintenance of expression vectors in C127 cells (see for example Howley et al., Meth. Enzymol. 101: 387-402, 1983).

The expression cassette may be included within a replication system for episomal maintenance in an appropriate cellular host or may be provided without a replication system, where it may become integrated into the host genome. The manner of transformation of the host organism with the various DNA constructs is not critical to this invention. The DNA may be introduced into the host in accordance with known techniques, such as transformation, using calcium phosphate precipitated DNA, electroporation, transfection by contacting the cells with a virus, microinjection of the DNA into cells or the like.

As a host organism, normal primary cells or cell lines derived from cultured primary tissue may be used, as well as microbial cells. The host cell is preferably a mammalian cell line, preferably hamster CHO cells, mouse C127 cells or human "293" cells, but microbial cells, for example yeast, preferably a Kluyveromyces species, or bacteria, preferably a Bacillus species, may also be used.

For stable transformation of a mammalian cell line with the expression vector bearing the Factor VIII congener coding sequence and subsequent amplification of the vector inserted in the host chromosome, Chinese hamster ovary cells (CHO) are especially suitable. Transformation involves transfection of the host cell with the expression vector together with a selectable marker gene such as dhfr or a G418 (neo-) resistance gene or the like, and subsequent integration into the chromosome and selection for stable transformation.

Another method for stable transformation for host cell lines uses expression vectors containing BPV-1 sequences. C127 cells are well suited for this purpose. Transformation involves transfection of the host cell with the expression vector containing BPV-1 sequences. Transformants are recognized by the formation of foci. Stable transformants are established and screened for Factor VIII activity using the Kabi Coatest according to examples 6 and 7. In contrast, when the expression is carried out in a transient transformation system such as COS-1 cells with pSV2-derived expression vectors, there is no selection step. Once the structural gene has been introduced into the appropriate host, the host may be grown to express the structural gene. The host cell may be grown to high density in an appropriate medium. Where the promoter is inducible, such as in a prokaryotic system, permissive conditions will then be employed, for example temperature change, exhaustion, or excess of a metabolic product or nutrient, or the like. In a mammalian system, where an amplifiable gene is used in tandem with the structural gene, the appropriate means for amplification will be employed.

Where secretion is provided for, the expression product, either fused or unfused, may be isolated from the growth medium by conventional means. Where secretion is not provided for, the host cells may be harvested and lysed in accordance with conventional methode. The desired product is then isolated and purified by conventional techniques, for example affinity chromatography with immobilized antibodies, chromatography on aminohexyl-sepharose or the mixed polyelectrolyte method.

The recombinant products may be glycosylated or non-glycosylated, having the wild-type or other glycosylation. The amount of glycosylation will depend in part upon the sequence of the particular peptide, as well as the organism in which it is produced. Thus, expression of the product in E. coli cells will result in an unglycosylated product, and expression of the product in mammalian cells will result in a product with glycosylation similar to the wild-type peptide.

### Uses of Factor VIII Congeners

The compounds can be used in a wide variety of ways, both in vivo and vitro. The compounds may be used as the active component of pharmaceutical preparations for treating patients exhibiting symptoms of Hemophilia A. By a pharmaceutical composition is meant any preparation to be administered to mammals. Thus, a pharmaceutical preparation with Factor VIII activity is a preparation which can be administered to a mammal to alleviate symptoms associated with Hemophilia A, the inability to properly clot blood. In preparing the pharmaceutical composition, generally the subject polypeptides are admixed with parenterally acceptable vehicles and other suitable excipients in accordance with procedures known in the art. The pharmaceutical preparations may conveniently be a sterile lyophilized preparation of the subject polypeptide which may be reconstituted by addition of a sterile solution suitable for producing solutions, preferably isotonic with the blood of the recipient. The pharmaceutical preparation may be presented in single unit or multi-dose containers, for example in sealed ampoules or vials. Their use would be analogous to that of known human Factor VIII polypeptide, appropriately adjusted for potency. The subject polypeptide may be administered in vivo, for example by injection, intravenously, peritoneally, subcutaneously, or the like.

The compounds additionally can be used for making antibodies to the subject compounds, which may find use in vivo or in vitro. The antibodies can be prepared in conventional ways, either by using the polypeptide as an immunogen and injecting the polypeptide into a mammalian host, for example mouse, cow, goat, sheep, rabbit, etc., particularly with an adjuvant, for example complete Freunds adjuvant, aluminum hydroxide gel, or the like. The most may then be bled and the blood employed for isolation of polyclonal antibodies, or in the cases of the mouse, the peripheral blood lymphocytes or splenic lymphocytes (B cells) employed for fusion with an appropriate myeloma cell to immortalize the chromosomes for monoclonal expression of antibodies specific for the compounds.

Either polyclonal or monoclonal antibodies may be prepared, which may then be used for diagnosis or detection of the presence of the polypeptide in a sample, such as cells or a physiological fluid, for example blood. Failure to detect the polypeptide may indicate the condition of hemophilia A.

Probes comprising sequences complementary to Factor VIII mRNA may also be prepared and used as a diagnostic aid, for example, the presence and/or amount of Factor VIII mRNA in a cell may be used for determining whether the patient is making Factor VIII but has developed antibodies which prevent its activity. A test sample comprising a cell, tissue sample or bodily fluid believed to contain hybridizing sequences can be treated so as to lyse any cells, then treated with a denaturing agent such as guanidine hydrochloride to release single-stranded mRNA. The probe labeled with, for example ³²P or biotinylated nucleotides, can then be hybridized to the cellular mRNA to form a double-stranded complex which may be detected by means of the label. For some purposes it may be desirable to quantitate the amount of Factor VIII mRNA. This is done by comparing the amount of label detected in reference samples containing known amounts of single-stranded Factor VIII mRNA with the amount of label detected in the test sample.

The following examples are offered by way of illustration and not by way of limitation.

### EXPERIMENTAL

General cloning techniques were used as described in Maniatis et al., Molecular Cloning: A Laboratory Manual, Cold Spring Harbor Laboratory, CSH, NY, 1982. All DNA-modifying enzymes were obtained from commercial suppliers. They were used according to manufacturers instructions. Materials and apparatus for DNA purification and separation were used according to instructions from the supplier.

### Example 1 (Reference)

### Construction of Expression Vector PCLB201

pCLB201 is an expression vector comprising the RSV-LTR promoter in the right orientation for transcriptional initiation, and the full-length Factor VIII cDNA (see Figure 2). It is derived from the pSV2-vector of Mulligan and Berg, Proc. Natl. Acad. Sci. USA (1981) 78:2072.

The unique HindIII site of the plasmid pSV2.tPA (Van Zonneveld et al., Proc. Natl. Acad. Sci. USA (1986) 83:4670-4674) was modified into a SalI site by making blunt the HindIII sticky-ends and ligating SalI-linkers (5'-CGTCGACG-3') to the blunt ends. A plasmid containing a SalI site was selected and identified as pCLB91. This plasmid is the same as pSV2.tPA except that it contains a SalI linker inserted into the HindIII site.

The isolation of Factor VIII mRNA from human liver, and the preparation, purification and identification of its cDNA and its assembly in the plasmid pEP121 resulting in plasmid pCLB89 have been described in patent application EP 0253455, which disclosure is hereby incorporated by reference. A 7440 bp long SalI-HpaI fragment from pCLB89 containing the factor VIII cDNA (see Figure 1) was purified and inserted into pCLB91 digested with SalI and BglII. The resulting expression vector pCLB200 contained an intact SalI site with the HpaI site ligated to the BglII site which had been filled in.

Plasmid pRSVneo (Gorman, DNA Cloning, 1985, ed., D. Glover, IRL-press, Washington, Oxford, pp. 143-169) was digested with NdeI and HindIII and incubated with the Klenow fragment of DNA polymerase to create blunt ends and the 0.6 kb fragment containing a Rous Sarcoma Virus-Long Terminal Repeat (RSV-LTR) sequence was isolated and inserted into the SalI site of pCLB200 which had similarly been made blunt-ended to form expression vector pCLB201 (see Figure 2).

### Example 2 (According to the Invention)

### Construction of pCLB203

### (A) pCLB100:

The SalI-HpaI 7440 bp fragment (see Figure 2) from pCLB89 was inserted into plasmid pSP64 (Melton et al., Nucleic Acids Res. (1984) 12:7035-7056) which had been cleaved with SalI and SmaI. The resulting plasmid, pCLB100, contained an intact SalI-site and the HpaI-end linked to the SmaI*-*end.

### (B) pCLB101: (numbers in parentheses refer to Figure 1)

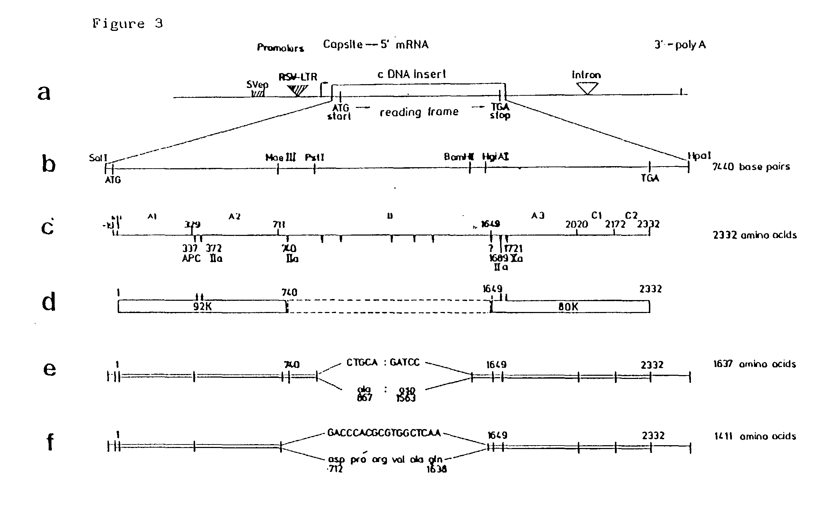

(1) Plasmid pCLB100 was digested with KpnI and Tth111I, generating a 631 bp fragment: KpnI (1817) to Tth111I (2447). The 631 bp fragment was purified, then out with MaeIII (2199). The MaeIII sticky end was filled in resulting in a 383 bp fragment.
(2) Plasmid pCLB100 was digested with ApaI and BanI. The 669 bp ApaI (6200)-BanI (5532) fragment was isolated.
(3) Plasmid pCLB100 was digested with HgiAI, producing a HgiAI-fragment of 634 bp. The fragment incubated with T4 DNA polymerase to make blunt ends, then digested with BanI, to produce a 565 bp fragment.
(4) Plasmid pCLB100 was digested with ApaI and KpnI, generating a 5,9 kb long fragment (ApaI (6200) to KpnI (1817)) containing vector sequences for maintenance and propagation in E. coli.
(5) The fragments obtained in steps (1) through (4) were purified and equimolar amounts of the fragments and a ten-fold molar excess of MluI-linker (CCACGCGTGG) were ligated, giving rise to plasmid pCLB101. Plasmid pCLB101 contained a MluI-linker between the MaeIII and HgiAI sites (see Figure 3b) in addition to KpnI, BanI and ApaI sites. Four extra amino acids (P-R-V-A) between amino acids D-712 and Q-1638 (Figure 1) are shown in Figure 3f.

### (C) pCLB203:

pCLB101 and pCLB201 were digested with enzymes KpnI and XbaI. The 2.4 kb KpnI-XbaI fragment from pCLB101 was ligated to the 7.0 kb KpnI-XbaI fragment derived from pCLB200. The resulting plasmid pCLB203 possessed intact KpnI and XbaI sites. Its structure is depicted in Figure 3f.

### Example 3

### Transient Expression of Recombinant Factor VIII DNA and Assay of Produced Proteins

### A. Transfection of COS-1 Cells and metabolic labeling

The expression vectors obtained according to the previous examples were introduced into COS-1 cells using a DEAE transfection technique. Vector DNA was precipitated by incubating with DEAE-dextran for 2 hrs, followed by treatment with chloroquine shock for 2 hrs, as described by Lopata et al., Nucleic Acids Res. 12, 5707, (1984) and Luthman and Magnusson, Nucleic Acids Res. (1983) 11:1295. The medium used for growth of the COS-1 cells and also for the conditioned medium was Iscove's DMEM (Flow) supplemented with 10% (v/v) heat inactivated fetal calf serum (Flow). The medium was changed 48 hrs after transfection. The conditioned medium was collected 48 hrs Later.

Metabolic labeling of the proteins in the transfected cells was carried out using serum free RPMI-medium (Gibco). Two days after transfection transfectants were incubated for 4 hrs with 50 uCi/ml L-³⁵S-methionine (Amersham, 1985; 370 MBeq/330 ul; specific activity: over 100 Ci/mMole), followed by an overnight incubation with 1 mM L-methionine before harvesting the conditioned medium. In order to suppress protein degradation, protease inhibitors such as phenyl methane sulphonyl fluoride (PMSF) were added to the conditioned medium after harvesting. To inhibit protein glycosylation, tunicamycin can be added to the conditioned medium (final concentration 0.001 mg/ml). Conditioned media were harvested 4 days after transfection; produced proteins were assayed.

### B. Biological Activity of Recombinant Factor VIII

The conditioned medium was assayed for Factor VIII activity using (1) the standard coagulation or clotting assay and (2) the chromogenic activity assay (Kabi Coatest).

The standard coagulation or clotting assay (so-called activated partial thromboplastin time) was performed using hemophilia plasma as described by Veldkamp et al., Thromb. Diath. Haemorrh. (1968) 19:279. The conditioned medium was citrated before examination.

The chromogenic activity or Kabi Coatest assay was performed according to procedures supplied by the manufacturer Kabi-Vitrum, except that all volumes prescribed were divided by four and 25 ul of conditioned medium was tested. The Factor VIII-like proteins were activated for 15 min, at which time the chromogenic substrate (S2222) was added.

Inhibition of Factor VIII activity was measured according to the standard Bethesda protocol (Kasper et al., Thromb. Diath. Haemorrh. (1975) 34:869-871. The immunoglobulins used were purified by ion exchange and protein A Sepharose chromatography prior to use.

The standard for biological activity assays for Factor VIII was a pool of citrated plasma (0.5 mM final conc.) which was assumed to contain 1 U of Factor VIII activity or antigen per ml.

The biological activity for various of the deletion proteins are shown in Table II. In addition, the established activity of the recombinant protein solution appeared to be inhibited following addition of antibodies known to be inhibitors of plasma-derived Factor VIII activity and/or of so-called inhibitor-sera, obtained from patients with inhibitors, i.e., antibodies against Factor VIII, in their blood.

### C. Immunologic Cross-reactivity with Factor VIII

### (1) Preparation of monoclonal antibodies.

Balb/c mice were immunized with purified human Factor VIII-von Willebrand factor complex obtained by agarose gel filtration of cryoprecipitate or a Factor VIII concentrate used for therapeutical purposes (Central Laboratory of the Netherlands Red Cross Blood Transfusion Service, Amsterdam, The Netherlands), Van Mourik and Mochtar, Biochim. Biophys. Acta 221:677-679 (1970). Lymphocyte hybridization was performed as described by Galfre et al., Nature (1977) 266:550-552. Description of the techniques used for selection of clones producing monoclonal antibodies to Factor VIII has been provided elsewhere (Stel, 1984, Ph.D. thesis, University of Amsterdam, The Netherlands). Monoclonal antibodies to Factor VIII were identified according to their reactivity with Factor VIII polypeptides as described in European patent application EP 0253455.

### (2) Polyclonal antibodies to Factor VIII polypeptides.

Rabbits were immunized by standard procedures with a Factor VIII preparation which had been purified by chromatography (Stel, supra). The antibodies thus obtained were identified by immunoblotting, as described in European patent application EP 0253455, using purified Factor VIII-von Willebrand factor or purified polypeptides. The antibodies were isolated by polyacrylamide gel electrophoresis then transferred to nitrocellulose sheets as target proteins. Three distinct polyclonal antisera were obtained: RH 63271, RH 63272 and RH 63275. The antisera reacted with the 80 kDa doublet, 92 kDa polypeptide and larger polypeptides. These antisera also reacted with smaller fragments of Factor VIII.

### (3) ELISA

A newly developed ELISA (Enzyme Linked Immuno-Sorbent Assay) was used to detect Factor VIII antigen in the conditioned medium. The method is as follows. The ELISA plates were coated by adding 200 ul/well of an appropriate dilution of the Factor VIII specific monoclonal antibody CLB.CAgA (5 mg/l) in 0.05 M carbonate buffer, pH 9.8. The plates were sealed and incubated overnight at 4°C. Between all incubations the plates were washed three times with PBS containing 0.05% Tween-20, which was left on the plates for at least one minute.

A series of dilutions of test samples or normal plasma was pipetted into the wells (200 ul/well) in duplicate. The plates were sealed and incubated at 37°C for 2 hrs without stirring, then washed as described above. For dilution of the antigen a buffer was used which contained 50 mM Tris-HCl buffer, 2% bovine serum albumin and 1.0 M sodium choride, pH 7.4.

CLB-CAgll7-horse radish peroxidase conjugates were diluted approximately 10,000-fold (depending on the sensitivity required) in a buffer containing 50 mM Tris-HCl buffer, 0.2% Tween-20 and 1.0 M sodium choride, pH 7.4. Of this dilution, 200 ul was added to each well, the plates were sealed and incubatedd for 2 hrs at 37°C in the dark.

After washing the plates as described above, 150 ul of a freshly prepared solution of tetramethylbenzidine (TMB) (0.1 g/l) and hydrogen peroxide (0.006%) in 0.1 M acetate/citric acid buffer, pH 5.5 was added to each well. The plates were incubated for 30 min in the dark at room temperature. The enzyme reaction was stopped by the addition of 150 ul 2N sulphuric acid. Adsorption was determined at 450 nm with an ELISA microreader. As shown in Table II there was an increase in Factor VIII activity of successive conditioned medium which was approximately proportional to the increase in the amount of Factor VIII proteins in the medium,

### D. Size determination

The size of the produced Factor VIII proteins was established using gel electrophoresis as follows.

The monoclonal and polyclonal sera raised against plasma-derived Factor VIII were used for immunoprecipitation, The antibodies were immobilized on protein A-Sepharose (15 ul serum per 10 mg protein A-Sepharose), then incubated with the metabolically labeled recombinant Factor VIII-like compounds. The immobilized recombinant proteins were reduced using 10% beta-mercaptoethanol, then separated on a 5% polyacrylamide SDS gel electrophoresis system (Laemmli, Nature (1972) 227:680-685). As shown in Figure 4 and Table III, the recombinant Factor VIII-like proteins have the size expected for glycosylated proteins. The smaller bands were also present in the control lane.

Based upon the results presented in Table III it can also be concluded that the recombinant Factor VIII-like proteins of the subject invention are glycosylated, since there is a significant difference between the size of the proteins formed in medium with and without tunicamycin, a known inhibitor of asparagine-linked glycosylation.

### Example 4

### Construction of Stable Cell Lines Producing Proteins with Factor VIII Activity

### A. Expression in CHO Cells

Plasmids pCLB203 (10 ug) and pAdD26SV(A)-3 (1 ug, Kaufman and Sharp, Mol. Cell Biol. (1982) 2:1304-1319) were introduced into dhfr-deficient CHO cells (Chasin and Urlaub, Proc. Natl. Acad. Sci. USA (198C) 77:4216-4220) using the calcium phosphate precipitation technique (Graham and Van der Eb, Virology (1973) 52:456-467). Amplification of Factor VIII and dhfr coding sequences was achieved by stepwise administration of increasing concentrations of methotrexate (mtx) as described by Kaufman and Sharp, J. Mol. Biol. (1982) 159:601-621. Independent transformants resistant to 200 nM mtx were picked and established as stable cell lines. Several of these cell lines produced about 75 mU of Factor VIII activity/ml of culture medium. The amount of Factor VIII activity secreted into the culture medium could be further raised by further amplification of the Factor VIII coding sequences, using mtx in increasing concentrations.

### B. Expression in C127 Cells

As described above, expression vectors may be introduced into eukaryotic cells, where they integrate into the genome of the host cell. Subsequently the expression cassettes may be amplified. An alternative to integration of the expression vector is its stable maintenance as an episome. An example of the latter is the expression of one of the "mutant" Factor VIII protein using the episomal BPV-system (Howley et al., Meth. Enzymol. (1983) 101:387-402). The BPV-1 genome (BamHI cleaved) was first introduced into a BamHI cleaved derivative of pTZ18R (Pharmacia) which contains XhoI sites on both sites of the BamHI site. Subsequently the resulting pTZX-BPV plasmid was cleaved using XhoI, yielding a 2.9 kb pTZ fragment and an 8 kb BPV-fragment with XhoI protruding ends. The latter fragment was ligated into plasmid pCLB212 which had been cleaved at its unique SalI site (at position 2040 in the original pSVL vector). The resulting expression vector pGB881 contains the SV40 late promoter, Factor VIII cDNA coding sequence lacking 2775 bp (mainly of the B-domain) and SV40 late polyadenylation signal. Due to the presence of the BPV-genome this vector can be maintained as an episome in the proper host cells, such as mouse C127 cells. Plasmid pCB881 (10 ug) was transfected into C127 cells using the calcium phosphate precipitation technique. (Graham and Van der Eb, supra). Foci were isolated 14 days after transfection and subsequently stable cell lines were established. Several cell lines produced 40 mU of Factor VIII activity/ml of culture medium.

### REFERENCES

Brinkhous, K.M. et al., (1985) Proc. Natl. Acad. Sci. USA 82, 8752-8756
Burke, R.L. et al., (1986) J. Biol. Chem. 261, 12574-12578.
Carter, P. et al., (1985) Nucleic Acids Res. 13, 4431
Chasin, L.A. and Urlaub, G. (1980) Proc. Natl. Acad. Sci. USA 77, 4216-4220
Eaton, D.L. et al., (1986a) Biochemistry 25, 505-512
Eaton, D.L. et al., (1986b) Biochemistry 25, 8343-8347
Fay, J.F. et al., (1986) Biochim. Biophys. Acta 871, 268-278
Galfré, G. et al., (1977) Nature 266, 550-552
Gluzman, Y., (1981) Cell 23, 175-182
Gorman, C., (1985) DNA cloning (Ed.: D. Glover), IRL-press, Washington, Oxford, pp. 143-169
Graham, F. and Van der Eb, A. (1973) Virology 52, 456-467
Hanahan, D., (1983) J. Mol. Biol. 166, 557-580
Howley, P.M., Sarver, N. and Law, M.F. (1983) Meth. Enzymol. 101, 387-402
Hurwitz, D.R., Hodges, R., Drohan, W. and Sarver, H. (1987) Nucl. Acids Res. 15, 7137-7153
Kasper, C.K. et al., (1975) Thrombs. Diath. Haemorrh. 34, 869-871
Kaufman, R.J. and Sharp, P.A. (1982) Mol. Cell. Biol. 2, 1304-1319
Kaufman, R.J. and Sharp, P.A. (1982a) J. Mol. Biol. 159, 601-621
Kozak, M. (1983) Microbiol. Rev. 47, 1-45
Kramer, W. et al., (1984) Nucleic Acids Res. 12, 9441-9456
Laemmli, U.K., (1970) Nature 227, 680-685
Luthman, H. and Magnusson, G. (1983) Nucleic Acids Res. 11, 1295
Maniatis, T. et al., (1982) Molecular Cloning. Cold Spring Harbor Laboratory
Maxam, A.M. amd Gilbert, W. (1980) Methods Enzymol. 65, 499-560
Melton, D.A. et al., (1984) Nucleic Acids Res. 12, 7035-7056
Mulligan, R. and Berg, P. (1981) Proc. Natl. Acad. Sci. USA, 78, 2072
Sanger, F. et al., (1977) Proc. Natl. Acad. Sci. USA 74, 5463-5467
Stel, H.V., (1984) Ph.D. thesis, University of Amsterdam, The Netherlands
Toole, J.T., et al., (1986) Proc. Natl. Acad. Sci. USA 83, 5939-5942
Van Mourik, J.A. and Mochtar, J.A., (1970) Biochim. Biophys. Acta 221, 677-679
Van Zonneveld, A.J., et al., (1986) Proc. Natl. Acad. Sci USA 83, 4670-4674
Vehar, G.A., et al., (1984) Nature 312, 327-342
Veldkamp, J.J. et al., (1968) Thromb. Diathes Haemorrh. 19, 279
Wood, W.I. et al., (1984) Nature 312, 330-337
Yanisch-Perron, C et al., (1985) Gene 33, 103-119

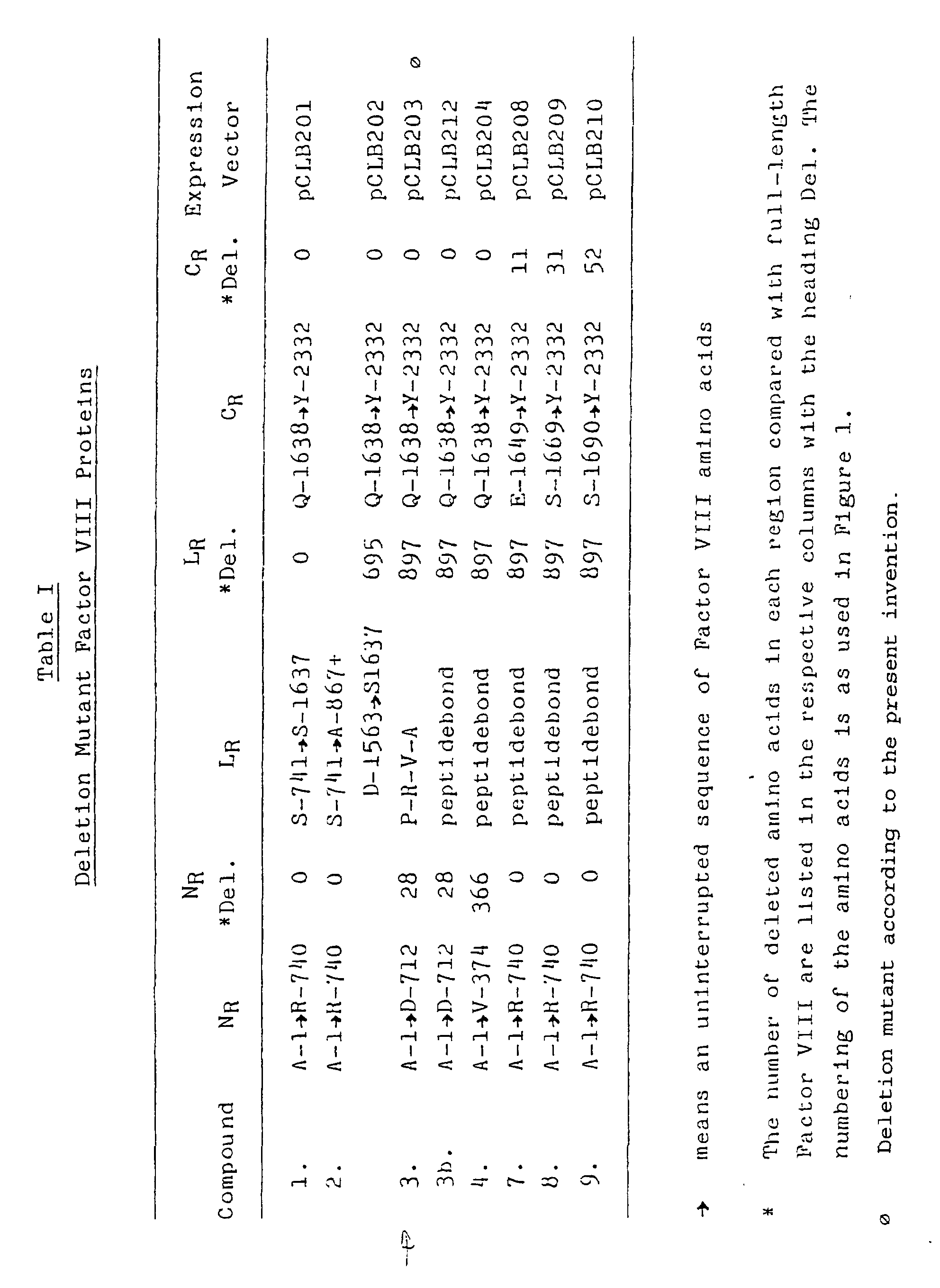

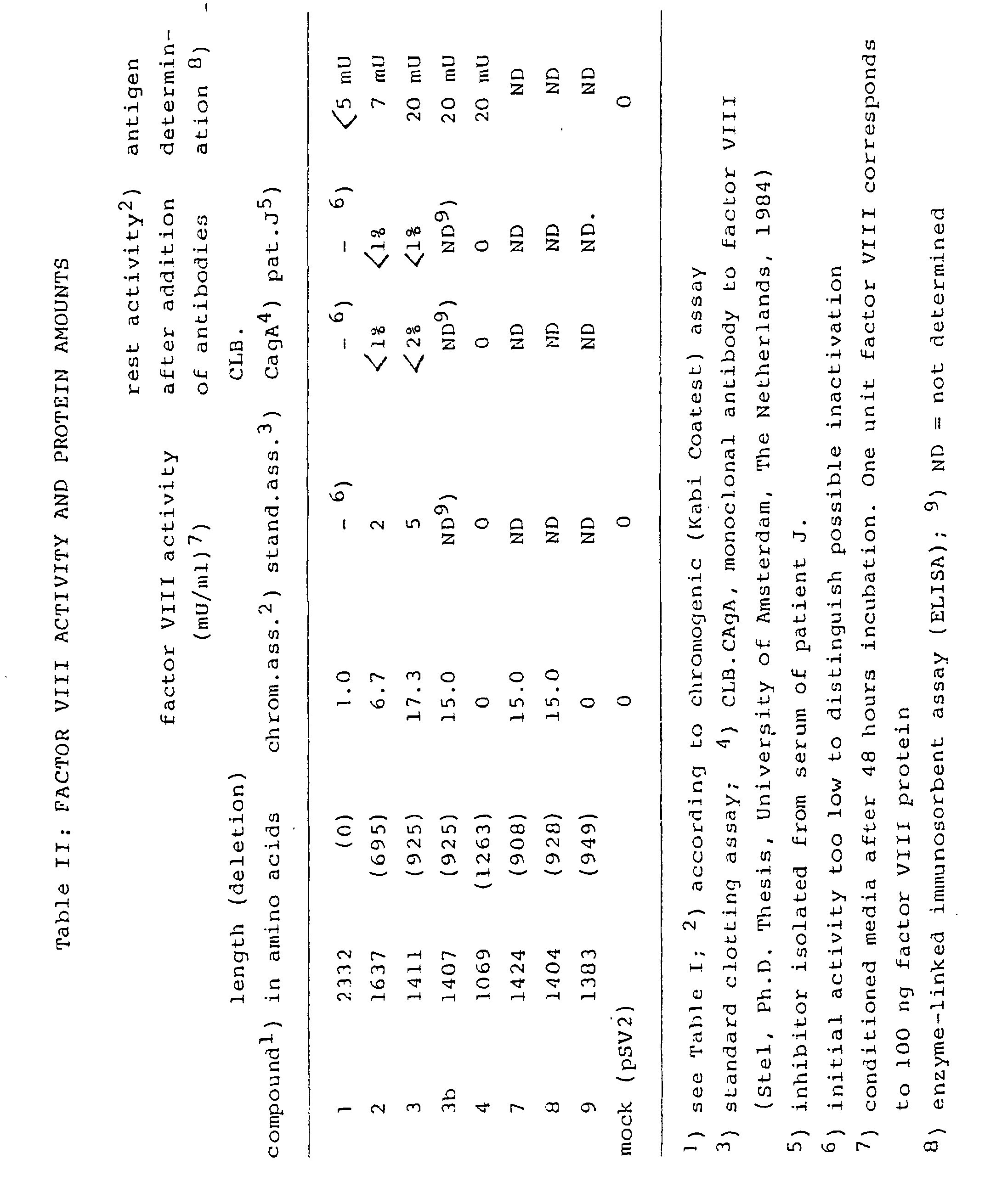

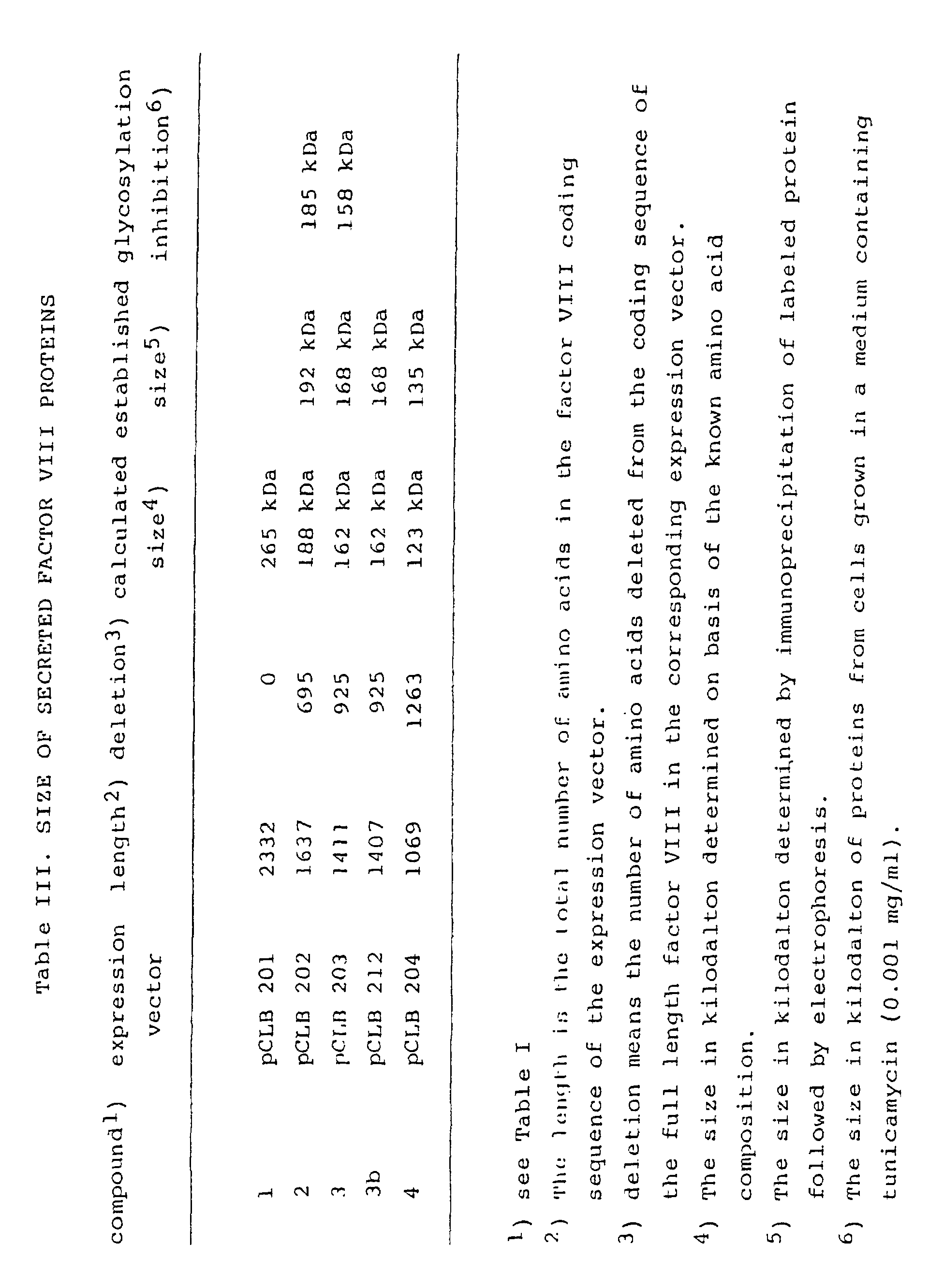

## Claims

1. A protein which has biological activity of Factor VIII and an amino acid sequence represented by the formula N_{R}-L_{R}-C_{R} wherein N_{R} represents an amino acid sequence corresponding substantially to amino acids Ala-1 through Asp-712 of Factor VIII;
L_{R} represents the amino acid sequence Pro-Arg-Val-Ala; and
C_{R} represents an amino acid sequence corresponding substantially to amino acids Gln-1638 through Tyr-2332 of Factor VIII.

2. A recombinant DNA molecule comprising a DNA sequence coding for a protein as defined in claim 1.

3. An expression vector comprising, in the direction of transcription, a transcriptional regulatory region and a translational initiation region functional in a host cell, a DNA sequence coding for a protein as defined in claim 1 and translational and transcriptional termination regions functional in said host cell, wherein expression of said DNA sequence is regulated by said initiation and termination regions.

4. A transformed cell and progeny thereof, said cell comprising an expression vector comprising, in the direction of transcription, a transcriptional regulatory region and a translational initiation region functional in a host cell, a DNA sequence coding for a protein as defined in claim 1 and translational and transcriptional termination regions functional in said host cell, wherein expression of said DNA sequence is regulated by said initiation and termination regions; but excluding any non-isolated human cell.

5. A transformed cell and progeny thereof according to claim 4, wherein said cell is a mammalian cell.

6. A transformed cell and progeny thereof according to claim 5, wherein said cell is a COS cell, a CHO cell or a C127 cell.

7. A transformed cell and progeny thereof according to claim 4, wherein said cell is a prokaryotic cell or a yeast cell.

8. A transformed cell and progeny thereof according to claim 7, wherein said cell is of a Bacillus or Kluyveromyces species.

9. A method for preparing a protein which is a deletion mutant of factor VIII having biological activity of Factor VIII, said method comprising growing, in a nutrient medium, a host cell comprising an expression vector comprising, in the direction of transcription, a transcriptional regulatory region and a translational initiation region functional in a host cell, a DNA sequence coding for a protein as defined in claim 1 and translational and transcriptional termination regions functional in said host cell, wherein expression of said DNA sequence is regulated by said initiation and termination regions, and isolating said protein.

10. A Factor VIII deficiency symptom-alleviating composition comprising a symptom-alleviating amount of a protein which is a deletion mutant of Factor VIII as defined in claim 1 having biological activity of Factor VIII, and a physiologically acceptable carrier.

## Patentansprüche

1. Protein mit biologischer Faktor VIII Aktivität und mit einer Aminosäuresequenz, entsprechend der Formel NR-LR-C_{R}, wobei N_{R} eine Aminosäuresequenz darstellt, die im wesentlichen den Aminosäuren Ala-1 bis Asp-712 von Faktor VIII entspricht, L_{R} die Aminosäuresequenz Pro-Arg-Val-Ala darstellt, und C_{R} eine Aminosäuresequenz darstellt, die im wesentlichen den Aminosäuren Gln-1638 bis Tyr-2332 von Faktor VIII entspricht.

2. Rekombinantes DNA-Molekül, umfassend eine DNA-Sequenz, welche ein Protein gemäß Anspruch 1 codiert.

3. Expressionsvektor, umfassend in Transkriptionsrichtung einen transkriptionalen regulatorischen Bereich und einen translationalen Initiationsbereich, die in einer Wirtszelle funktional sind, eine DNA-Sequenz, die ein Protein nach Anspruch 1 codiert und translationale und transkriptionale Terminationsbereiche, die in der Wirtszelle funktional sind, worin die Expression der DNA-Sequenz durch die Initiations und Terminationsbereiche reguliert wird.

4. Transformierte Zelle und Abkömmlinge davon, wobei die Zelle einen Expressionsvektor enthält, der, in Transkriptionsrichtung einen transkriptionalen regulatorischen Bereich und einen translationalen Initiationsbereich, die in der Wirtszelle funktional sind, eine DNA-Sequenz, die ein Protein gemäß Anspruch 1 codiert, und translationale und transkriptionale Terminationsbereiche, die in der Wirtszelle funktional sind, umfasst, worin die Expression der DNA-Sequenz, durch die Initiations- und Terminationsbereiche reguliert wird, ausgenommen einer nicht isolierten menschlichen Zelle.

5. Transformierte Zelle und Abkömmlinge davon nach Anspruch 4. worin die Zelle eine Säugerzelle ist.

6. Transformierte Zelle und Abkömmlinge davon nach Anspruch 5, worin die Zelle eine COS-Zelle, eine CHO-Zelle oder eine C127-Zelle ist.

7. Transformierte Zelle und Abkömmlinge davon nach Anspruch 4, worin die Zelle eine prokaryontische oder eine Hefezelle ist.

8. Transformierte Zelle und Abkömmlinge davon nach Anspruch 7, worin die Zelle zur Gattung *Bacillus* oder Kluyveromyces gehört.

9. Verfahren zur Herstellung eines Proteins, welches eine Deletionsmutante des Faktors VIII mit biologischer Aktivität des Faktors VIII ist, wobei das Verfahren das Wachsen lassen in einem Nährmedium von einer wirtszelle, die einen Expressionvektor enthält, der in Transkriptionsrichtung, einen transkriptionalen regulatorischen Bereich und einen translationalen Initiationsbereich, die in der wirtszelle funktional sind, eine DNA-Sequenz, codierend ein Protein nach Anspruch 1, und translationale und transkriptionale Terminationsbereiche, die in der Wirtszelle funktional sind, umfasst, worin die Expression der DNA-Sequenz durch die Initiations- und Terminationsbereiche reguliert wird, und Isolieren des Proteins umfasst.

10. Faktor VIII-Mangelsymptom-lindernde Zusammensetzung, umfassend eine Symptom-lindernde Menge des Proteins, welches eine Deletionsmutante des Faktors VIII. wie in Anspruch 1 definiert ist mit biologischer Aktivität von Paktor VIII und einen physiologisch annehmbaren Träger.

## Revendications

1. Protéine à activité biologique du facteur VIII et ayant une séquence d'amino-acides représentée par la formule N_{R}-L_{A}-C_{R} dans laquelle N_{R} représente une séquence d'amino-acides correspondant substantiellement aux amino-acides Ala-1 à Asp-712 du facteur VIII ;
L_{R} représente la séquence d'amino-acide's Pro-Arg-Val-Ala ; et
C_{R} représente une séquence d'amino-asides correspondant substantiellement aux amino-acides Gln-1638 à Tyr-2332 du facteur VIII,

2. Molécule d'ADN recombinant comprenant une séquence d'ADN codant pour une protéine répondant à la définition suivant la revendication 1.

3. Vecteur d'expression comprenant, dans le sens de la transcription, une région de régulation de transcription et une région d'initiation de traduction fonctionnelles dans une cellule hôte, une séquence d'ADN codant pour une protéine répondant à la définition suivant la revendication 1 et des régions de terminaison de traduction et de transcription fonctionnelles dans ladite cellule hôte, dans lequel l'expression de ladite séquence d'ADN est régulée par lesdites régions d'initiation et de terminaison.

4. Cellule transformée et sa descendance, ladite cellule comprenant un vecteur d'expression renfermant, dans le- sens de la transcription, une région de régulation de transcription et une région d'initiation de traduction fonctionnelles dans une cellule hôte, une séquence d'ADN codant pour une protéine répondant à la définition suivant la revendication 1, et des régions de terminaison et de transcription fonctionnelles dans ladite cellule hôte, 'dans laquelle l'expression de ladite séquence d'ADN est régulée par lesdites régions d'initiation et de tenninaison ; mais à l'exclusion de toute cellule humaine non isolée.

5. Cellule transformée et sa descendance suivant la revendication 4, ladite cellule étant une cellule de mammifère.

6. Cellule transformée et sa descendance suivant la revendication 5, ladite cellule étant une cellule COS, une cellule CHO ou une cellule C127.

7. Cellule transformée et sa descendance suivant la revendication 4, ladite cellule étant une cellule procaryotique ou une cellule de levure.

8. Cellule transformée et sa descendance suivant la revendication 7, ladite cellule étant une cellule d'une espèce de Bacillus ou de Kluyveromyces.

9. Procédé pour la préparation d'une protéine qui est *un* mutant par délétion du facteur VIII ayant l'activité biologique du facteur VIII, ledit procédé comprenant la culture, dans un milieu nutritif, d'une cellule hôte comprenant un vecteur d'expression renfermant, dans le lens de la transcription, une région de régulation de transcription et une région d'initiation de traduction fonctionnelles dans une cellule hôte, une séquence d'ADN codant pour une protéine répondant à la définition suivant la revendication 1 et des régions de terminaison de traduction et de transcription fonctionnelles dans ladite cellule hôte, dans lequel l'expression de ladite séquence d'ADN est régulée par lesdites régions d'initiation et de terminaison, et l'isolement de ladite protéine.

10. Composition réduisant les symptômes de déficience en facteur VIII, comprenant une quantité, ayant pour effet de réduire les symptômes, d'une protéine qui est un mutant par délétion du facteur VIII répondant à la définition suivant la revendication 1, ayant l'activité biologique du facteur VIII, et un support physiologiquement acceptable.
